Europäisches Patentamt

European Patent Office

Office européen des brevets

(11) Veröffentlichungsnummer: **0 591 716 B1**

(12) **EUROPÄISCHE PATENTSCHRIFT**

(45) Veröffentlichungstag der Patentschrift: **08.11.95**

(51) Int. Cl.6: **C09J 4/06**, A61K 6/00

(21) Anmeldenummer: **93114787.0**

(22) Anmeldetag: **15.09.93**

(54) **Konditionierungsmittel und Verfahren zur Vorbehandlung von Kunststoff-Oberflächen und Verwendung des Konditionierungsmittels.**

(30) Priorität: **08.10.92 DE 4233886**

(43) Veröffentlichungstag der Anmeldung:
**13.04.94 Patentblatt 94/15**

(45) Bekanntmachung des Hinweises auf die
Patenterteilung:
**08.11.95 Patentblatt 95/45**

(84) Benannte Vertragsstaaten:
**BE CH DK FR GB IT LI NL**

(56) Entgegenhaltungen:
**EP-A- 0 068 696
EP-A- 0 407 059
EP-A- 0 436 205
EP-A- 0 452 540**

(73) Patentinhaber: **HERAEUS KULZER GMBH
Heraeusstrasse 12-14
D-63450 Hanau (DE)**

(72) Erfinder: **Eykmann, Rolf
Auf der Feldwiese 8a
D-61273 Wehrheim (DE)**
Erfinder: **Erdrich, Albert, Dr.
Wiesbadener Strasse 48
D-61350 Bad Homburg (DE)**
Erfinder: **Hohmann, Alfred
Hornauer Strasse 43b
D-65779 Kelkheim (DE)**

(74) Vertreter: **Kühn, Hans-Christian
Heraeus Holding GmbH,
Stabsstelle Schutzrechte,
Heraeusstrasse 12-14
D-63450 Hanau (DE)**

# EP 0 591 716 B1

## Beschreibung

Die Erfindung betrifft ein polymerisierbares Konditionierungsmittel auf Methacrylat-Basis und ein Verfahren zur Vorbehandlung der Oberfläche von Formkörpern aus Polyacrylat-, Polymethacrylat- und Polycarbonat-Kunststoffen vor dem Auftragen von polymerisierbarem Methacrylat-Material und die Verwendung des Konditionierungsmittels.

Ein photopolymerisierbares Material auf Methacrylat-Basis, das sich als Klebstoff beziehungsweise Bindemittel zum Verbinden von Teilen aus Acrylat-Kunststoffen miteinander oder mit noch nicht ausgehärtetem monomerem Methacrylat-Material eignet, ist aus EP 0 142 172 A2 bekannt. Neben einem Initiator für die Photopolymerisation enthält es eine polare organische Verbindung, besonders Acrylsäure oder Methacrylsäure, vernetzend wirkendes Dimethacrylat, ein Methacrylat (Dicyclopentenyloxyäthylmethacrylat, Methylmethacrylat) oder Methylenchlorid als Verdünnungs- beziehungsweise Lösungsmittel und gegebenenfalls noch ein Urethandimethacrylat. Es wird besonders bei der Herstellung von Zahnprothesen, um die aus Acrylat-Kunststoff bestehenden künstlichen Zähne mit dem die Prothesenplatte bildenden Methacrylat-Material zu verbinden, und für die Reparatur beschädigter Zahnprothesen verwendet.

In DE 40 00 171 A1 wird ein lichthärtendes Klebmittel beschrieben, das neben Methylmethacrylat, einem Methylmethacrylat-Polymer und einem Photoinitiator ein mehrfunktionelles Acrylat oder Methacrylat, vorzugsweise ein Tri- oder Tetraacrylat beziehungsweise -methacrylat, in einer Menge von 1-20 Gewicht-% enthält. Das Klebmittel ist zum Verbinden von Formkörpern aus Acrylat-Kunststoff mit anderen Kunststofformkörpern geeignet, wobei wenigstens einer der beteiligten Kunststofformkörper für die zur Aushärtung verwendete Strahlung ausreichend durchlässig sein muß.

DE 40 12 720 A1 betrifft einen Klebstoff zum Verbinden von Formteilen aus Polycarbonat-Kunststoffen miteinander, der durch ein Peroxid/Amin-System als Katalysator für die Kaltpolymerisation ausgehärtet wird. Der Klebstoff enthält neben dem Peroxid/Amin-System Alkylmethacrylat, 2,2-Bis-[4-(methacryloyloxyalkoxy)-phenyl]-propan, Dimethacrylat eines Alkandiols- und/oder Trimethacrylat eines Alkantriols und Polymethylmethacrylat und liegt zunächst in Form zweier Komponenten vor, die vor Gebrauch miteinander vermischt werden müssen.

Aus EP 0 476 789 A1 ist ein orthodontisches Bracket bekannt, das aus Füllstoff-haltigem Methacrylatkunststoff besteht und auf der an dem natürlichen Zahn mit Hilfe eines Klebstoffs zu befestigenden Oberfläche eine dünne Schicht aus in dem Klebstoff teilweise löslichem oder quellbarem Methacrylatkunststoff trägt. Die dünne Methacrylatkunststoff-Schicht wird durch Auftragen und Polymerisieren einer Mischung aus A) 30-70 Gewichts-% eines monofunktionellen Methacrylats, 30-70 Gewichts-% eines Methacrylat-Homo- oder -Copolymers und 0,01-1 Gewichts-% eines Polymerisationskatalysators oder B) 5-24 Gewichts-% eines mehrfunktionellen Methacrylats, 0-24 Gewichts-% eines monofunktionellen Methacrylats, 50-90 Gewichts-% eines Siliciumdioxid-Füllstoffs und 0,01-1 Gewichts-% eines Polymerisationskatalysators erhalten.

Es ist die Aufgabe der Erfindung, ein polymerisierbares Mittel - im folgenden als Konditionierungsmittel bezeichnet - für die Vorbehandlung der Oberfläche von Polyacrylat-, Polymethacrylat- und Polycarbonat-Kunststofformkörpern zur Erzeugung einer festen Verbindung dieser Formkörper mit Methacrylat-Material zu finden. Das Methacrylat-Material liegt zunächst in polymerisierbarer Form vor und wird nach dem Aufbringen auf die mit dem Konditionierungsmittel vorbehandelte Kunststoff-Oberfläche durch Polymerisation ausgehärtet. Das Konditionierungsmittel soll besonders zur Vorbehandlung konfektionierter Kunststoff-Zähne und fertiger Zahnprothesen auf Polymethacrylat-Basis und orthodontischer Brackets auf Polycarbonat-Basis dienen. Eine Vorbehandlung der konfektionierten Zähne und der Zahnprothesen ist dann erforderlich, wenn sie zum Beispiel durch Aufbringen von gefärbtem Methacrylat-Material den individuellen farblichen Gegebenheiten im Munde des Patienten angepaßt werden sollen, die der Brackets, um ihre mit Hilfe eines Klebstoffs erfolgende Befestigung an den natürlichen Zähnen zu verbessern.

Ein weiteres Ziel der Erfindung ist es, ein Verfahren zur Vorbehandlung der Oberfläche von Polyacrylat-, Polymethacrylat- und Polycarbonat-Kunststofformkörpern mit einem polymerisierbaren Konditionierungsmittel auf Methacrylat-Basis vor dem Aufbringen von polymerisierbarem Methacrylat-Material zu finden, mit dem sich eine feste und dauerhafte Bindung zwischen den Kunststofformkörpern und dem durch Polymerisation ausgehärteten Methacrylat-Material erzeugen läßt. Das Verfahren soll sich besonders zur Vorbehandlung konfektionierter Kunststoff-Zähne und fertiger Zahnprothesen auf Polymethacrylat-Basis, wenn diese hinsichtlich der Farbe und Form den individuellen Gegebenheiten beim Patienten anzupassen sind, und orthodontischer Brackets auf PolycarbonatBasis vor ihrer mit Hilfe eines Klebstoffs erfolgenden Befestigung an den natürlichen Zähnen eignen.

Das die Lösung der Aufgabe darstellende Konditionierungsmittel ist erfindungsgemäß dadurch gekennzeichnet, daß es 50-75 Gewichts-% Alkylmethacrylat oder ein Gemisch aus Alkylmethacrylat und Butandiol-

2

dimethacrylat mit mindestens 50 Gewichts-% Alkylmethacrylat, 15-40 Gewichts-% Diurethandimethacrylat aus 2-Hydroxyethylmethacrylat und 2,2,4-Trimethylhexamethylendiisocyanat, 1-15 Gewichts-% Polymethylmethacrylat, 0-5 Gewichts-% Trimethacrylat, Tetramethacrylat oder ein Gemisch davon, 0,01-1 Gewichts-% monocyclischen Terpenkohlenwasserstoff und 0,1-5 Gewichts-% Polymerisationskatalysator enthält.

Besonders bewährt hat sich das Konditionierungsmittel, das 60-70 Gewichts-% Alkylmethacrylat oder ein Gemisch aus Alkylmethacrylat und Butandioldimethacrylat mit mindestens 50 Gewichts-% Alkylmethacrylat, 20-30 Gewichts-% Diurethandimethacrylat aus 2-Hydroxyethylmethacrylat und 2,2,4-Trimethylhexamethylendiisocyanat, 2-10 Gewichts-% Polymethylmethacrylat, 1-3 Gewichts-% Trimethacrylat, Tetramethacrylat oder ein Gemisch daraus, 0,1-0,5 Gewichts-% monocyclischen Terpenkohlenwasserstoff und 0,5-3 Gewichts-% Polymerisationskatalysator enthält.

Bevorzugt wird das Konditionierungsmittel, das als Alkylmethacrylat Methylmethacrylat und/oder Ethylmethacrylat und als Trimethacrylat, falls vorhanden, Trimethylolpropantrimethacrylat enthält; ein geeignetes Tetramethacrylat ist zum Beispiel Di-trimethylol-propantetramethacrylat. Als monocyclischen Terpenkohlenwasserstoff enthält es vorzugsweise Terpinolen.

Das Polymethylmethacrylat kann bei der Zubereitung des Konditionierungsmittels als solches, vorzugsweise in Form von Polymerisat-Perlen mit einer Teilchengröße von 10-150 Mikrometer, oder als Lösung in dem Alkylmethacrylat, vorzugsweise in Methylmethacrylat, eingesetzt werden. Ein Polymethylmethacrylat mit einem mittleren Molekulargewicht von 120 000 - 200 000 wird dabei bevorzugt.

Der in dem Konditionierungsmittel enthaltene Polymerisationskatalysator löst durch Radikalbildung eine radikalische Polymerisation des Konditionierungsmittels aus und ist vorzugsweise ein solcher für die Kaltpolymerisation (chemische Polymerisation) oder für die Photopolymerisation. Für die Kaltpolymerisation besonders geeignete Katalysatoren sind die bekannten Redox-Systeme, vorzugsweise solche aus organischen Peroxiden und Aminen, zum Beispiel Dibenzoylperoxid/N,N-Dimethyl-p-toluidin. Als Katalysatoren für die Photopolymerisation haben sich Keton/Amin-Systeme, zum Beispiel Campherchinon/Amin (GB 1 408 265), und Acylphosphinoxid-Verbindungen (DE 28 30 927 A1), gegebenenfalls im Gemisch mit einem α-Hydroxyacetophenon, besonders bewährt. Die Anwendung von Photopolymerisationskatalysatoren bietet die Möglichkeit, das Konditionierungsmittel als ein alle Bestandteile enthaltendes gebrauchsfertiges Einkomponenten-Material zur Verfügung zu haben. Dagegen liegt das ein Peroxid/Amin-System enthaltende Konditionierungsmittel zunächst in Form zweier Komponenten vor, deren erste das Peroxid und deren zweite das Amin enthält. Vor Gebrauch werden beide Komponenten miteinander vermischt.

Das Konditionierungsmittel kann neben den genannten Bestandteilen kleine Mengen üblicher Zusätze, wie zum Beispiel Pigmente, Stabilisatoren, Inhibitoren und Antioxidantien, enthalten.

Die Lösung der Aufgabe besteht weiterhin in einem Verfahren zur Vorbehandlung der Oberfläche von Formkörpern aus Polyacrylat-, Polymethacrylat- und Polycarbonat-Kunststoffen mit einem polymerisierbaren Konditionierungsmittel auf Methacrylat-Basis vor dem Auftragen von polymerisierbarem Methacrylat-Material, das erfindungsgemäß dadurch gekennzeichnet ist, daß die Kunststoff-Oberfläche mit einer dünnen Schicht eines Konditionierungsmittels, das 50-75 Gewichts-% Alkylmethacrylat oder ein Gemisch aus Alkylmethacrylat und Butandioldimethacrylat mit mindestens 50 Gewichts-% Alkylmethacrylat, 15-40 Gewichts-% Diurethandimethacrylat aus 2-Hydroxyethylmethacrylat und 2,2,4-Trimethylhexamethylendiisocyanat, 1-15 Gewichts-% Polymethylmethacrylat, 0-5 Gewichts-% Trimethacrylat, Tetramethacrylat oder ein Gemisch davon, 0,01-1 Gewichts-% monocyclischen Terpenkohlenwasserstoff und 0,1-5 Gewichts-% Polymerisationskatalysator enthält, versehen und anschließend das Konditionierungsmittel polymerisiert wird.

Besonders bewährt hat sich das Verfahren, wenn die Kunststoff-Oberfläche mit einer dünnen Schicht des Konditionierungsmittels, das 60-70 Gewichts-% Alkylmethacrylat oder ein Gemisch aus Alkylmethacrylat und Butandioldimethacrylat mit mindestens 50 Gewichts-% Alkylmethacrylat, 20-30 Gewichts-% Diurethandimethacrylat aus 2-Hydroxyethylmethacrylat und 2,2,4-Trimethylhexamethylendiisocyanat, 2-10 Gewichts-% Polymethylmethacrylat, 1-3 Gewichts-% Trimethacrylat, Tetramethacrylat oder ein Gemisch daraus, 0,1-0,5 Gewichts-% monocyclischen Terpenkohlenwasserstoff und 0,5-3 Gewichts-% Polymerisationskatalysator enthält, versehen wird.

Die Art und Weise der Polymerisation des Konditionierungsmittels hängt von dem in ihm enthaltenen Polymerisationskatalysator ab, der entweder ein Katalysator für die Kaltpolymerisation (chemische Polymerisation) oder die Photopolymerisation ist. Bei der Photopolymerisation wird die Bestrahlung mit sichtbarem Licht bevorzugt.

Besonders bewährt hat sich das Verfahren, wenn die Kunststoff-Oberfläche mit einer 0,01-0,3 Millimeter dicken Schicht des Konditionierungsmittels versehen wird.

Die unter Anwendung des Konditionierungsmittel und nach dem Verfahren gemäß der Erfindung hergestellten Kombinationen aus Polyacrylat-, Polymethacrylatbeziehungsweise Polycarbonat-Kunststofformkörpern und polymerisiertem Methacrylat-Material zeichnen sich durch ihren dichten, randspaltenfreien, gegenüber Wasser und Temperaturwechsel-Belastung beständigen und dauerhaften Verbund aus. Bei der Anwendung des Konditionierungsmittel und des Verfahrens auf dem Dentalgebiet erweist sich die Beständigkeit des Verbundes gegenüber den Bedingungen im Munde als besonders vorteilhaft.

Zur näheren Erläuterung werden in den folgenden Beispielen einige bevorzugte Ausführungsformen des Konditionierungsmittels und des Verfahrens gemäß der Erfindung und im Anschluß daran die Prüfung der damit erhaltenen Kombinationen aus Kunststofformkörper und polymerisiertem Methacrylat-Material anhand von Festigkeitsuntersuchungen beschrieben. Die in den Beispielen als Diurethandimethacrylat bezeichnete Verbindung ist das Reaktionsprodukt aus 2-Hydroxyethylmethacrylat (2 mol) und 2,2,4-Trimethylhexamethylendiisocyanat (1 mol). 2,6-Di-tert.-butyl-4-methylphenol dient als Stabilisator.

EP 0 591 716 B1

Beispiele 1 – 7

Konditionierungsmittel

1) Methylmethacrylat      65,88 Gewichts-%
Diurethandimethacrylat      26 Gewichts-%
Polymethylmethacrylat      5,4 Gewichts-%
Terpinolen      0,2 Gewichts-%
2,4,6-Trimethylbenzoyl-diphenylphosphinoxid      2,5 Gewichts-%
2,6-Di-tert.-butyl-4-methylphenol      0,02 Gewichts-%

2) Methylmethacrylat      67,28 Gewichts-%
Diurethandimethacrylat      20 Gewichts-%
Polymethylmethacrylat      7,5 Gewichts-%
Trimethylolpropantrimethacrylat      2,5 Gewichts-%
Terpinolen      0,2 Gewichts-%
2,4,6-Trimethylbenzoyl-diphenylphosphinoxid      2,5 Gewichts-%
2,6-Di-tert.-butyl-4-methylphenol      0,02 Gewichts-%

3) Methylmethacrylat      38,78 Gewichts-%
Butandioldimethacrylat      25 Gewichts-%
Diurethandimethacrylat      25 Gewichts-%
Polymethylmethacrylat      6 Gewichts-%
Trimethylolpropantrimethacrylat      2,5 Gewichts-%
Terpinolen      0,2 Gewichts-%
2,4,6-Trimethylbenzoyl-diphenylphosphinoxid      2,5 Gewichts-%
2,6-Di-tert.-butyl-4-methylphenol      0,02 Gewichts-%

4) Ethylmethacrylat      54,78 Gewichts-%
Methylmethacrylat      12,3 Gewichts-%
Diurethandimethacrylat      25 Gewichts-%
Polymethylmethacrylat      5,2 Gewichts-%
Terpinolen      0,2 Gewichts-%
2,4,6-Trimethylbenzoyl-diphenylphosphinoxid      2,5 Gewichts-%
2,6-Di-tert.-butyl-4-methylphenol      0,02 Gewichts-%

5

5)  Methylmethacrylat                                        65,98 Gewichts-%

    Diurethandimethacrylat                                   26    Gewichts-%

    Polymethylmethacrylat                                    5,4   Gewichts-%

    Terpinolen                                               0,1   Gewichts-%

    2,3-Bornandion (Campherchinon)                           1     Gewichts-%

    2-Butoxyethyl-(4-dimethylaminobenzoat)                   1,5   Gewichts-%

    2,6-Di-tert.-butyl-4-methylphenol                        0,02  Gewichts-%


6)  Methylmethacrylat                                        68,18 Gewichts-%

    Diurethandimethacrylat                                   26    Gewichts-%

    Polymethylmethacrylat                                    5,4   Gewichts-%

    Terpinolen                                               0,1   Gewichts-%

    9,10-Phenanthrenchinon                                   0,1   Gewichts-%

    N,N-Bis-(2-hydroxyethyl)-p-toluidin                      0,2   Gewichts-%

    2,6-Di-tert.-butyl-4-methylphenol                        0,02  Gewichts-%


7)  Methylmethacrylat                                        65,88 Gewichts-%

    Diurethandimethacrylat                                   26    Gewichts-%

    Polymethylmethacrylat                                    5,4   Gewichts-%

    Terpinolen                                               0,2   Gewichts-%

    2,4,6-Trimethylbenzoyl-diphenylphosphinoxid              1,25  Gewichts-%

    1-(4-Isopropylphenyl)-2-hydroxy-2-methylpropanon         1,25  Gewichts-%

    2,6-Di-tert.-butyl-4-methylphenol                        0,02  Gewichts-%


Beispiel 8

Individuelle Anpassung eines konfektionierten Kunststoff-Zahnes

Ein Zahn aus Kunststoff auf Polymethacrylat-Basis (Vitapan Vita Zahnfabrik, Bad Säckingen) wird oberflächlich durch Sandstrahlen mit Aluminiumoxid-Pulver aufgerauht und anschließend gereinigt. Dann wird das Konditionierungsmittel nach Beispiel 1 in dünner Schicht (etwa 0,1 Millimeter dick) auf die Oberfläche aufgetragen und der so behandelte Zahn zur Photopolymerisation der Konditionierungsmittel-Schicht dem Licht (Xenon-Blitzlampe) des Dentacolor® XS-Lichthärtegeräts (Heraeus Kulzer GmbH, Hanau) ausgesetzt, wobei die Polymerisationszeit 90 Sekunden beträgt. Danach wird der Zahn unter Verwendung der Hals-, Dentin- und Schmelzmasse des photopolymerisierbaren Dentacolor® Kronen- und Brückenmaterials (Composite mit mikrofeinem Füllstoff, Heraeus Kulzer GmbH, Hanau) und von photopolymerisierbaren Malfarben in Form und Farbe den ihn umgebenden natürlichen Zähnen angepaßt und zur Polymerisation des Kronen- und Brückenmaterials und der Malfarben erneut dem Licht des Dentacolor® XS-Lichthärtegeräts ausgesetzt; die Polymerisationszeit beträgt wiederum 90 Sekunden. Der so hergestellte individualisierte Kunststoff-Zahn wird dann noch nachgearbeitet und poliert.

Beispiel 9

Befestigung eines orthodontischen Brackets an einem natürlichen Zahn

Die zur Befestigung an dem natürlichen Zahn vorgesehene Oberfläche eines orthodontischen Brackets aus Polycarbonat-Kunststoff wird durch Sandstrahlen mit Aluminiumoxid-Pulver aufgerauht, gereinigt und dann mit einer dünnen Schicht (etwa 0,05 Millimeter dick) des Konditionierungsmittels nach Beispiel 1 versehen. Zur Photopolymerisation des Konditionierungsmittels wird das so behandelte Bracket dem Licht des Dentacolor® XS-Lichthärtegeräts (Heraeus Kulzer GmbH, Hanau) ausgesetzt; die Polymerisationszeit beträgt dabei 90 Sekunden. Dann wird mit Microfill pontic (Befestigungscomposite mit mikrofeinem Füllstoff, Heraeus Kulzer GmbH, Hanau) das Bracket auf dem mit einer Säure angeätzten natürlichen Zahn befestigt.

Beispiel 10

Individuelles Anpassen einer Kunststoff-Zahnprothese

Eine Kunststoff-Zahnprothese aus Polymethacrylat-Kunststoff wird an den den Gegebenheiten im Munde des Patienten anzupassenden Stellen oberflächlich durch Sandstrahlen mit Aluminiumoxid-Pulver aufgerauht, gereinigt und dann mit einer dünnen Schicht (etwa 0,2 Millimeter dick) des Konditionierungsmittels nach Beispiel 1 versehen. Zur Photopolymerisation des Konditionierungsmittels wird die so behandelte Zahnprothese dem Licht des Dentalcolor® XS-Lichthärtegeräts (Heraeus Kulzer GmbH, Hanau) ausgesetzt; die Polymerisationszeit beträgt dabei 90 Sekunden. Dann erfolgt unter Verwendung der Hals- Dentin-, Schmelz- und Zahnfleischmasse des photopolymerisierbaren Dentacolor® Kronen- und Brückenmaterials (Composite mit mikrofeinem Füllstoff, Heraeus Kulzer GmbH, Hanau) und von photopolymerisierbaren Malfarben die gewünschte Form- und Farbanpassung sowohl der das Zahnfleisch imitierenden Teile als auch der künstlichen Zähne der Prothese und anschließend die Polymerisation des Kronenund Brückenmateriels und der Malfarben in dem Dentacolor® Lichthärtegerät XS. Die so hergestellte individualisierte Kunststoff-Zahnprothese wird noch nachgearbeitet und poliert.

Beispiele 11 - 22

Festigkeits- und Feuchtebeständigkeitsprüfung

Die Beurteilung des Konditionierungsmittels und des Verfahrens gemäß der Erfindung erfolgt durch Prüfung der Festigkeitseigenschaften von aus konfektionierten Kunststoff-Zähnen entsprechend dem Beispiel 8 hergestellten individualisierten Kunststoff-Zähnen, wobei in den Beispielen 11-14 das Konfektionierungsmittel nach Beispiel 1 und - zum Vergleich dazu - in den Beispielen 15-18 ein im Handel erhältlicher photopolymerisierbarer Haftvermittler aus monomeren Methacrylsäureestern und Initiator für die Photopolymerisation (Special-Bond 2, Vivadent, Ellwangen) und in den Beispielen 19-22 ein alle Bestandteile außer Terpinolen des in Beispiel 1 beschriebenen Konditionierungsmittels enthaltendes Mittel und handelsübliche Zähne auf Polymethacrylat-Basis mit der Bezeichnung Bioplus (DeTrey/Dentsply, Dreieich), Vitapan (Vita Zahnfabrik, Bad Säckingen), Vivodent (Ivoclar, Schaan, Liechtenstein) und Vivosit (Ivoclar, Schaan, Liechtenstein) verwendet werden. Die Prüfung der Festigkeitseigenschaften der individualisierten Kunststoff-Zähne umfaßt die Bestimmung der Verbundfestigkeit im Scherversuch (Scherfestigkeit [MPa])
a) nach 24 Stunden langer Lagerung in Wasser von 37 ° C,
b) nach 5000 Temperaturwechsellast-Cyclen, 5 ° C/55 ° C ("thermal cycling") und
c) nach 4 Wochen langer Lagerung in Wasser von 37 ° C.
Die nach 24 Stunden langer Lagerung in Wasser von 37 ° C (a) bestimmten Scherfestigkeitswerte werden in der Tabelle I, die nach der Temperaturwechsel-Belastung (b) und nach 4 Wochen langer Lagerung in Wasser von 37 ° C (c) in der Tabelle II angegeben.

EP 0 591 716 B1

Tabelle I

| Beispiel | Scherfestigkeit [MPa] a |
|---|---|
| 11[1] | 15,6 |
| 12[2] | 16,5 |
| 13[3] | 17,0 |
| 14[4] | 21,5 |
| 15[1] (Vgl.) | 10,7 |
| 16[2] (Vgl.) | 13,1 |
| 17[3] (Vgl.) | 12,2 |
| 18[4] (Vgl.) | 13,0 |
| 19[1] (Vgl.) | 11,7 |
| 20[2] (Vgl.) | 12,5 |
| 21[3] (Vgl.) | 12,8 |
| 22[4] (Vgl.) | 15,0 |

[1]Bioplus
[2]Vitapan
[3]Vivodent
[4]Vivosit

Tabelle II

| Beispiel | Scherfestigkeit [MPa] | | |
|---|---|---|---|
| | a | b | c |
| 11[1] | 15,6 | 14,5 | 14,5 |
| 12[2] | 16,5 | 14,2 | 18,2 |
| 13[3] | 17,0 | 15,1 | 16,4 |
| 14[4] | 21,5 | 15,6 | 18,6 |

[1]Bioplus
[2]Vitapan
[3]Vivodent
[4]Vivosit

**Patentansprüche**

1. Polymerisierbares Konditionierungsmittel auf Methacrylat-Basis zur Vorbehandlung der Oberfläche von Formkörpern aus Polyacrylat-, Polymethacrylat- und Polycarbonat-Kunststoffen vor dem Auftragen von polymerisierbarem Methacrylat-Material, dadurch gekennzeichnet, daß es 50-75 Gewichts-% Alkylmethacrylat oder ein Gemisch aus Alkylmethacrylat und Butandioldimethacrylat mit mindestens 50 Gewichts-% Alkylmethacrylat, 15-40 Gewichts-% Diurethandimethacrylat aus 2-Hydroxyethylmethacrylat und 2,2,4-Trimethylhexamethylendiisocyanat, 1-15 Gewichts-% Polymethylmethacrylat, 0-5 Gewichts-% Trimethacrylat, Tetramethacrylat oder ein Gemisch davon, 0,01-1 Gewichts-% monocyclischen Terpenkohlenwasserstoff und 0,1-5 Gewichts-% Polymerisationskatalysator enthält.

2. Konditionierungsmittel nach Anspruch 1, dadurch gekennzeichnet, daß es 60-70 Gewichts-% Alkylmethacrylat oder ein Gemisch aus Alkylmethacrylat und Butandioldimethacrylat mit mindestens 50 Gewichts-% Alkylmethacrylat, 20-30 Gewichts-% Diurethandimethacrylat aus 2-Hydroxyethylmethacrylat und 2,2,4-Trimethylhexamethylendiisocyanat, 2-10 Gewichts-% Polymethylmethacrylat, 1-3 Gewichts-% Trimethacrylat, Tetramethacrylat oder ein Gemisch daraus, 0,1-0,5 Gewichts-% monocyclischen Terpenkohlenwasserstoff und 0,5-3 Gewichts-% Polymerisationskatalysator enthält.

8

3.  Konditionierungsmittel nach Anspruch 1 oder 2, dadurch gekennzeichnet, daß es als Alkylmethacrylat Methylmethacrylat und/oder Ethylmethacrylat enthält.

4.  Konditionierungsmittel nach einem der Ansprüche 1 bis 3, dadurch gekennzeichnet, daß es als Trimethacrylat Trimethylolpropantrimethacrylat enthält.

5.  Konditionierungsmittel nach einem der Ansprüche 1 bis 4, dadurch gekennzeichnet, daß es als monocyclischen Terpenkohlenwasserstoff Terpinolen enthält.

6.  Verfahren zur Vorbehandlung der Oberfläche von Formkörpern aus Polyacrylat-, Polymethacrylat- und Polycarbonat-Kunststoffen mit einem polymerisierbaren Konditionierungsmittel auf Methacrylat-Basis vor dem Auftragen von polymerisierbarem Methacrylat-Material, dadurch gekennzeichnet, daß die Kunststoff-Oberfläche mit einer dünnen Schicht eines Konditionierungsmittels, das 50-75 Gewichts-% Alkylmethacrylat oder ein Gemisch aus Alkylmethacrylat und Butandioldimethacrylat mit mindestens 50 Gewichts-% Alkylmethacrylat, 15-40 Gewichts-% Diurethandimethacrylat aus 2-Hydroxyethylmethacrylat und 2,2,4-Trimethylhexamethylendiisocyanat, 1-15 Gewichts-% Polymethylmethacrylat, 0-5 Gewichts-% Trimethacrylat, Tetramethacrylat oder ein Gemisch davon, 0,01-1 Gewichts-% monocyclischen Terpenkohlenwasserstoff und 0,1-5 Gewichts-% Polymerisationskatalysator enthält, versehen und anschließend das Konditionierungsmittel polymerisiert wird.

7.  Verfahren nach Anspruch 6, dadurch gekennzeichnet, daß die Kunststoff-Oberfläche mit einer dünnen Schicht des Konditionierungsmittels, das 60-70 Gewichts-% Alkylmethacrylat oder ein Gemisch aus Alkylmethacrylat und Butandioldimethacrylat mit mindestens 50 Gewichts-% Alkylmethacrylat, 20-30 Gewichts-% Diurethandimethacrylat aus 2-Hydroxyethylmethacrylat und 2,2,4-Trimethylhexamethylendiisocyanat, 2-10 Gewichts-% Polymethylmethacrylat, 1-3 Gewichts-% Trimethacrylat, Tetramethacrylat oder ein Gemisch daraus, 0,1-0,5 Gewichts-% monocyclischen Terpenkohlenwasserstoff und 0,5-3 Gewichts-% Polymerisationskatalysator enthält, versehen wird.

8.  Verfahren nach Anspruch 6 oder 7, dadurch gekennzeichnet, daß die Kunststoff-Oberfläche mit einer dünnen Schicht des Konditionierungsmittels, das als Alkylmethacrylat Methylmethacrylat und/oder Ethylmethacrylat enthält, versehen wird.

9.  Verfahren nach einem der Ansprüche 6 bis 8, dadurch gekennzeichnet, daß die Kunststoff-Oberfläche mit einer dünnen Schicht des Konditionierungsmittels, das als Trimethacrylat Trimethylolpropantrimethacrylat enthält, versehen wird.

10. Verfahren nach einem der Ansprüche 6 bis 9, dadurch gekennzeichnet, daß die Kunststoff-Oberfläche mit einer dünnen Schicht des Konditionierungsmittels, das als monocyclischen Terpenkohlenwasserstoff Terpinolen enthält, versehen wird.

11. Verfahren nach einem der Ansprüche 6 bis 10, dadurch gekennzeichnet, daß die Kunststoff-Oberfläche mit einer 0,01-0,3 Millimeter dicken Schicht des Konditionierungsmittels versehen wird.

12. Verfahren nach einem der Ansprüche 6 bis 11, dadurch gekennzeichnet, daß das Konditionierungsmittel durch Bestrahlung mit Licht polymerisiert wird.

13. Verwendung des Konditionierungsmittels nach einem der Ansprüche 1 bis 5 bei der individuellen Anpassung von konfektionierten Kunststoff-Zähnen und Zahnprothesen auf Polymethacrylat-Basis mit Hilfe von polymerisierbarem Methacrylat-Material.

14. Verwendung des Konditionierungsmittels nach einem der Ansprüche 1 bis 5 bei der Befestigung von orthodontischen Brackets aus Polycarbonat-Kunststoff an natürlichen Zähnen mit Hilfe von polymerisierbarem Methacrylat-Material.

**Claims**

1.  A polymerisable conditioning medium based on methacrylate for the pretreatment of the surface of shaped bodies of polyacrylate-, polymethacrylate- and polycarbonate plastics before the application of polymerisable methacrylate material, characterised in that it contains 50-75 % by weight alkylmethacrylate or a mixture of alkylmethacrylate and butane diol dimethacrylate with at least 50% by weight alkylmethacrylate, 15-40 % by weight diurethane dimethacrylate of 2-hydroxyethylmethacrylate and 2,2,4-trimethylhexamethylene diisocyanate, 1-15 % by weight polymethylmethacrylate, 0-5 % by weight trimethacrylate, tetramethacrylate or a mixture thereof, 0.01-1 % by weight monocyclic terpene hydrocarbon and 0.1-5 % by weight polymerisation catalyst.

2. A conditioning medium according to Claim 1, characterised in that it contains 60-70 % by weight alkylmethacrylate or a mixture of alkylmethacrylate and butane diol dimethacrylate with at least 50 % by weight alkylmethacrylate, 20-30 % by weight diurethane dimethacrylate of 2-hydroxyethylmethacrylate and 2,2,4-trimethylhexamethylene diisocyanate, 2-10 % by weight polymethylmethacrylate, 1-3 % by weight trimethacrylate, tetramethacrylate or a mixture thereof, 0.1-0.5 % by weight monocyclic terpene hydrocarbon and 0.5-3 % by weight polymerisation catalyst.

3. A conditioning medium according to Claim 1 or 2, characterised in that it contains as alkylmethacrylate methylmethacrylate and/or ethylmethacrylate.

4. A conditioning medium according to one of Claims 1 to 3, characterised in that it contains as trimethacrylate trimethylol propane trimethacrylate.

5. A conditioning medium according to one of Claims 1 to 4, characterised in that it contains terpinolene as monocyclic terpene hydrocarbon.

6. A method for the pretreatment of the surface of shaped bodies of polyacrylate-, polymethacrylate- and polycarbonate plastics with a polymerisable conditioning medium based on methacrylate before the application of polymerisable methacrylate material, characterised in that the plastic surface is provided with a thin layer of a conditioning medium which contains 50-75 % by weight alkylmethacrylate or a mixture of alkylmethacrylate and butane diol dimethacrylate with at least 50 % by weight alkylmethacrylate, 15-40 % by weight diurethane dimethacrylate of 2-hydroxyethylmethacrylate and 2,2,4-trimethylhexamethylene diisocyanate, 1-15 % by weight polymethylmethacrylate, 0-5 % by weight trimethacrylate, tetramethacrylate or a mixure thereof, 0.01-1 % by weight monocyclic terpene hydrocarbon and 0.1-5 % by weight polymerisation catalyst, and then the conditioning medium is polymerised.

7. A method according to Claim 6, characterised in that the plastic surface is provided with a thin layer of the conditioning medium, which contains 60-70 % by weight alkylmethacrylate or a mixture of alkylmethacrylate and butane diol dimethacrylate with at least 50 % by weight alkylmethacrylate, 20-30 % by weight diurethane dimethacrylate of 2-hydroxyethylmethacrylate and 2,2,4-trimethylhexamethylene diisocyanate, 2-10 % by weight polymethylmethacrylate, 1-3 % by weight trimethacrylate, tetramethacrylate or a mixture thereof, 0.1-0.5 % by weight monocyclic terpene hydrocarbon and 0.5-3 % by weight polymerisation catalyst.

8. A method according to Claim 6 or 7, characterised in that the plastic surface is provided with a thin layer of the conditioning medium, which contains methylmethacrylate and/or ethylmethacrylate as alkylmethacrylate.

9. A method according to one of Claims 6 to 8, characterised in that the plastic surface is provided with a thin layer of the conditioning medium, which contains trimethylol propane trimethacrylate as trimethacrylate.

10. A method according to one of Claims 6 to 9, characterised in that the plastic surface is provided with a thin layer of the conditioning medium, which contains terpinolene as monocyclic terpene hydrocarbon.

11. A method according to one of Claims 6 to 10, characterised in that the plastic surface is provided with a 0.01-0.3 millimetre thick layer of the conditioning medium.

12. A method according to one of Claims 6 to 11, characterised in that the conditioning medium is polymerised by irradiation with light.

**13.** The use of the conditioning medium according to one of Claims 1 to 5 in the individual adjustment of manufactured plastic teeth and teeth prostheses based on polymethacrylate with the aid of polymerisable methacrylate material.

**14.** The use of the conditioning medium according to one of Claims 1 to 5 in the securing of orthodontic brackets of polycarbonate plastic to natural teeth with the aid of polymerisable methacrylate material.

**Revendications**

**1.** Agent de conditionnement polymérisable à base de méthacrylate destiné au traitement préalable de la surface de corps moulés en matières plastiques de type polyacrylate, polyméthacrylate et polycarbonate avant le dépôt d'un matériau de méthacrylate polymérisable, caractérisé en ce qu'il contient 50 à 75% en poids de méthacrylate d'alkyle ou d'un mélange de méthacrylate d'alkyle et de diméthacrylate de butanediol comportant au moins 50% en poids de méthacrylate d'alkyle, 15 à 40% en poids de diméthacrylate de diuréthanne provenant de méthacrylate de 2-hydroxyéthyle et de diisocyanate de 2,2,4-triméthylhexaméthylène, 1 à 15% en poids de poly(méthacrylate de méthyle), 0 à 5% en poids de triméthacrylate, de tétraméthacrylate ou d'un mélange de ces deux substances, 0,01 à 1% en poids d'hydrocarbure monocyclique terpénique et 0,1 à 5% en poids de catalyseur de polymérisation.

**2.** Agent de conditionnement selon la revendication 1, caractérisé en ce qu'il contient 60 à 70% en poids de méthacrylate d'alkyle ou d'un mélange de méthacrylate d'alkyle et de diméthacrylate de butanediol comportant au moins 50% en poids de méthacrylate d'alkyle, 20 à 30% en poids de diméthacrylate de diuréthanne provenant de méthacrylate de 2-hydroxyéthyle et de diisocyanate de 2,2,4-triméthylhexaméthylène, 2 à 10% en poids de poly(méthacrylate de méthyle), 1 à 3% en poids de triméthacrylate, de tétraméthacrylate ou d'un mélange de ces deux substances, 0,1 à 0,5% en poids d'hydrocarbure monocyclique terpénique et 0,5 à 3% en poids de catalyseur de polymérisation.

**3.** Agent de conditionnement selon la revendication 1 ou 2, caractérisé en ce qu'il contient du méthacrylate de méthyle et/ou du méthacrylate d'éthyle en tant que méthacrylate d'alkyle.

**4.** Agent de conditionnement selon une des revendications 1 à 3, caractérisé en ce qu'il contient du triméthacrylate de triméthylolpropane en tant que triméthacrylate.

**5.** Agent de conditionnement selon une des revendications 1 à 4, caractérisé en ce qu'il contient du terpinolène en tant qu'hydrocarbure monocyclique terpénique.

**6.** Procédé de traitement préalable de la surface de corps moulés en matières plastiques de type polyacrylate, polyméthacrylate et polycarbonate par un agent de conditionnement polymérisable à base de méthacrylate avant le dépôt d'un matériau de méthacrylate polymérisable, caractérisé en ce que la surface de matière plastique est enduite d'une couche mince d'agent de conditionnement qui contient 50 à 75% en poids de méthacrylate d'alkyle ou d'un mélange de méthacrylate d'alkyle et de diméthacrylate de butanediol comportant au moins 50% en poids de méthacrylate d'alkyle, 15 à 40% en poids de diméthacrylate de diuréthanne provenant de méthacrylate de 2-hydroxyéthyle et de diisocyanate de 2,2,4-triméthylhexaméthylène, 1 à 15% en poids de poly(méthacrylate de méthyle), 0 à 5% en poids de triméthacrylate, de tétraméthacrylate ou d'un mélange de ces deux substances, 0,01 à 1% en poids d'hydrocarbure monocyclique terpénique et 0,1 à 5% en poids de catalyseur de polymérisation, et que l'agent de conditionnement est ensuite polymérisé.

**7.** Procédé selon la revendication 6, caractérisé en ce que la surface de matière plastique est enduite d'une couche mince d'agent de conditionnement qui contient 60 à 70% en poids de méthacrylate d'alkyle ou d'un mélange de méthacrylate d'alkyle et de diméthacrylate de butanediol comportant au moins 50% en poids de méthacrylate d'alkyle, 20 à 30% en poids de diméthacrylate de diuréthanne provenant de méthacrylate de 2-hydroxyéthyle et de diisocyanate de 2,2,4-triméthylhexaméthylène, 2 à 10% en poids de poly(méthacrylate de méthyle), 1 à 3% en poids de triméthacrylate, de tétraméthacrylate ou d'un mélange de ces deux substances, 0,1 à 0,5% en poids d'hydrocarbure monocyclique terpénique et 0,5 à 3% en poids de catalyseur de polymérisation.

**8.** Procédé selon la revendication 6 ou 7, caractérisé en ce que la surface de matière plastique est enduite d'une couche mince d'agent de conditionnement qui contient du méthacrylate de méthyle et/ou du méthacrylate d'éthyle en tant que méthacrylate d'alkyle.

**9.** Procédé selon une des revendications 6 à 8, caractérisé en ce que la surface de matière plastique est enduite d'une couche mince d'agent de conditionnement qui contient du triméthacrylate de triméthylolpropane en tant que triméthacrylate.

**10.** Procédé selon une des revendications 6 à 9, caractérisé en ce que la surface de matière plastique est enduite d'une couche mince d'agent de conditionnement qui contient du terpinolène en tant qu'hydrocarbure monocyclique terpénique.

**11.** Procédé selon une des revendications 6 à 10, caractérisé en ce que la surface de matière plastique est enduite d'une couche d'agent de conditionnement de 0,01 à 0,3 mm d'épaisseur.

**12.** Procédé selon une des revendications 6 à 11, caractérisé en ce que l'agent de conditionnement est polymérisé par exposition à la lumière.

**13.** Utilisation de l'agent de conditionnement selon une des revendications 1 à 5 lors de l'adaptation individuelle de dents en matière plastique fabriquées industriellement et de prothèses dentaires à base de polyméthacrylate, au moyen d'un matériau de méthacrylate polymérisable.

**14.** Utilisation de l'agent de conditionnement selon une des revendications 1 à 5, lors de la fixation d'appareils orthodontiques en matière plastique de type polycarbonate à des dents naturelles au moyen d'un matériau de méthacrylate polymérisable.